# EUROPEAN PATENT APPLICATION

(11) **EP 1 506 773 A1**
(43) Date of publication of application: **16.02.2005**
(21) Application number: 04254862.8
(22) Date of filing: 12.08.2004
(51) Int. Cl.: A61K 7/48, A61K 7/40

(54) **Sprayable skin protectant compositions**

(30) Priority: 12.08.2003 US 639087
(71) Applicant: JOHNSON & JOHNSON CONSUMER COMPANIES, INC., Skillman, NJ 08558 (US)
(72) Inventor: Bussey, Deborah, Hamilton, NJ 08690 (US); Kaminski, Claudia, Milford, NJ 08848 (US); Santora, Delores, Hillsborough, NJ 08844 (US); Librizzi, Joseph, Hillsborough, NJ 08844 (US)
(74) Representative: Mercer, Christopher Paul

(57) **Abstract**

A skin-protectant composition containing a suspending agent, a rinse-off resistant agent; and at least one skin protectant agent, wherein the composition is substantially free of oil and silicone and is sprayable. Such compositions are suitable for treating the skin from, for example, diaper rash.

## Description

### Background of the Invention

### 1. Field of the Invention

The present invention relates to topical compositions, and more particularly topical skin protectant compositions that are sprayable.

### 2. Description of the Prior Art

Various skin protectant compounds are known for treating and/or protecting the skin in connection with indications such as minor burns; cuts; sunburn; scrapes; cracked or windburned skin; weeping and/or oozing of skin caused by poison sumac, poison oak, and/or poison ivy; and perineal dermatitis, which includes diaper dermatitis or "diaper rash."

Perineal dermatitis has been defined as contact dermatitis in the perineal area, including the perineum buttocks, and the perineal, coccyx, and upper/inner thigh regions. See Brown, D.S, et al., 39(7) A Conceptual Framework, Ostomy/Wound Management 20-25 (1993)("Brown"). The physical signs of diaper dermatitis may include one or a combination of erythema, oozing, selling, crusting, scaling, and visiculation, with the possibility of hyperpigmentation, thickening, and excoriation over time. See Brown.

Diaper dermatitis is believed to be caused by the prolonged contact of the skin with fecal matter and urine. Although the exact component or components of urine and feces responsible for diaper dermatitis has not been identified, some possible factors include ammonia, urine pH, fecal microorganisms, and lipase and protease enzymes found in fecal matter.

Currently, the main focus of dermatitis treatments has been to reduce the exposure of the skin to such body wastes via barrier products, e.g. diaper creams and lotions. Another mode of treatment also focuses on the incorporation of agents to inhibit various fecal enzymes, such as lipase, that often aggravate perineal dermatitis. See, e.g., W099/26619, WO 97/38735, USP 6,207,596 B1, and US 20030104019A1.

Disadvantageously, many current diaper rash ointments are difficult to apply due to their viscous consistency. In addition, because irritated areas of the skin are sore and sensitive to the touch, the application of such ointments directly on the skin makes the condition even more unpleasant, uncomfortable, and even painful. Further, such products are often messy and difficult to remove from the hands. It would be desirable to apply such products to the irritated area without the need to apply with the hand, such as via a sprayable form.

Many known diaper rash treatments incorporate solid active agents. In order to produce a sprayable formulation, these agents are often suspended with oils and/or silicones. See, e.g., US 2003/008223. However, there are safety concerns associated with the incorporation of such ingredients in sprayable treatments due to the potential inhalation of oils and/or silicones by the infants. When such oils and/or silicones are omitted from the formulations, the resulting products are often unstable and/or lack the water resistancy and barrier properties necessary for an effective skin protectant.

Therefore, there is a need for a safe and effective skin protectant composition that not only may be applied to the skin via a spray, but also retains sufficient water resistancy and barrier properties while on the skin.

### Summary of the Invention

In accordance with this invention, there is provided a skin protectant composition comprised of, consisting of, and/or consisting essentially of:
a. a suspending agent;
b. a rinse-off resistant agent; and
c. at least one skin protectant agent,
wherein the composition is substantially free of oil and silicone.

Another aspect of this invention is directed to a method of treating diaper rash in a human comprising applying the composition as described above to human skin.

The invention also provides for a hand-held spray pump dispenser containing and for spraying the compositions as described above.

We have unexpectedly discovered a composition that is effective, safe, and can be sprayed onto the skin without further irritating the skin with one's hands.

### Detailed Description of Preferred Embodiments

The term, "substantially free of oils and silicones," as used herein, shall mean that the composition contains, based upon the total weight of the composition, less than about 1.0 percent, e.g., less than about 0.5 percent or less than about 0.25 percent, of oils such as, for example, mineral oil, and silicones such as, for example, dimethicone.

By "skin protectant" it is meant the compounds that treat and/or protect the skin in connection with indications such as minor burns; cuts; sunburn; scrapes; cracked or windburned skin; weeping and/or oozing of skin caused by poison sumac, poison oak, and/or poison ivy; and perineal dermatitis, which includes diaper dermatitis or "diaper rash." In one embodiment, the compositions of the present invention are useful in the treatment of enzymatic dermatitis, such as perineal dermatitis, of the external skin. By "treat" or "treatment" is meant herein the reduction of a skin indication, e.g., dermatitis or the rash of the skin, or at least the stabilizing of the skin indication, or the prevention of such indications on the skin.

By "liquid" is meant that at ambient pressure and a temperature of 20 °C, the substance at issue has a continuous liquid phase before being sprayed.

By "affected area" is meant the area of skin that is presently or may be exhibiting any levels of an indication that may need treatment with a skin protectant, e.g. a skin rash or enzymatic dermatitis, or the area that will be in prolonged contact with feces containing such dermatitis-causing enzymes. It is the area at which treatment is desired. This area also includes the area immediately proximate to the described area.

By "suitable adherence to the skin" it is meant that after the composition of the present invention is applied to the skin, it will remain adhered to the skin for a period of time under normal use conditions to have an efficacious effect, e.g. for treating diaper rash.

The compositions of the present invention contain, based upon the total weight of the composition, from about 0.1 percent to about 5 percent, e.g. from about 0.1 percent to about 2 percent or from about 0.5 percent to about 1 percent of a suspending agent; from about 0.1 percent to about 10 percent, e.g. from about 1 percent to about 5 percent or from about 0.1 percent to about 5 percent of a rinse-off resistant agent; and a skin protectant agent.

The first component of the composition of the present invention is a suspending agent in the form of a swellable clay or adduct thereof. By the term "swellable clay," it is meant a clay having weakly bound ions in interlayer positions that may be hydrated or may absorb organic solvents. These clays generally possess a low cationic or anionic charge, i.e. less than about 0.9 units of charge per unit cell.

By the term "adducts," it is meant the oil swellable clays, i.e. those that swell in organic, non-aqueous solvents such as polar and nonpolar solvents, that may be prepared by reacting a water swellable clay with an organic material that binds to the clay. Examples of such binding organic materials include, but are not limited to, a quaternary ammonium compound having the structure:

R₁R₂R₃R₄N+ X-

wherein
R₁, R₂, R₃ and R₄ are each independently selected from H, a C₁ to C₂₂ alkyl, a C₁ to C₂₂ alkenyl, and a C₁ to C₂₂ aralkyl, provided that at least one of the R groups is such an alkyl, alkenyl or aralkyl; and
X is the water swellable clay.

The swellable clays useful in the present invention may be structured, or may be a mixture of both structured components and amphorous components. As used herein, the terms "amorphous clay" shall include any clay without an ordered structure. Examples of such amorphous clays include, but are not limited to, allophane, imogolite, and mixtures thereof.

Examples of various structures that may be present in the clay include sheets or layers, wherein a combination of such layers is referred to as a lattice structure. Examples of suitable clay lattice structures include the pyrophillite (dioctahedral) type, the talc (trioctahedral) type, or mixtures thereof. Classes of suitable structured swellable clays include, but are not limited to the smectite clays, sepiolite clays, zeolite clays, palygorskite clays, or mixtures thereof. Examples of suitable smectite clays that are useful in this invention include, but are not limited to, the aluminosilicate clays such as bentonite, montmorillonite, hectorite, sucinite, saponite, nontronite, vermiculite, beidellite, stevensite, and their synthetically made counterparts and mixtures thereof. Montmorillonite clay is preferred. See United States Patent No. 5,869,033 and US 20030104019A1, which are incorporated by reference herein.

In one embodiment, the clays may possess a multi-layer structure,
wherein at least one layer is comprised of a smectite clay or a mixture thereof. Other clays that may be either mixed with the smectite clay in a given layer or may comprise the other layers include, but are not limited to, sepiolite, palygorskite, zeolites, and mixtures thereof.

The second component of the composition of the present invention is a rinse-off resistant agent, which can be any known agent that enables the skin protectant agent to have a suitable adherence to the skin, e.g. the agents, when used alone or in a composition, pass the rinse-off resistant test and/or the transfer resistance test of Examples 3 and 4, respectively. Examples of suitable rinse-off resistant agents include, but are not limited to, maleic anyhydride terpolymers such as C30-38 olefin isopropyl maleate/maleic anhydride copolymer, which is commercially available from New Phase Technologies under the tradename, "Performa V 1608;" polyvinylpyrrolidone copolymers such as vinylpyrrolidone/eicosene copolymer, which is commercially available from International Specialty Products under the tradename, "Ganex V-220;" and copolymers and mixtures thereof.

The third component of the composition is an ingredient for protecting the skin, which includes but is not limited to oatmeal, soy, mica, silica, titanium dioxide, hydrocortisone, benzoyl peroxide, topical starch, zinc oxide, aluminum hydroxide gel, allantoin, cocoa butter, zinc acetate, calamine, glycerin, kaolin, talc, zinc carbonate, and mixtures thereof.

The concentration of the active ingredient(s) for protecting the skin will depend upon a number of factors such as, for example, the active ingredient selected and the result desired. However, the composition typically contains, based upon the total weight of the composition, at least about 0.001 percent, e.g. at least about 0.01 percent, at least about 0.1 percent, at least about 1 percent, at least about 5 percent or at least about 10 percent of such active ingredient(s). In embodiments wherein the composition is used to treat diaper rash, the compositions may contain, based upon the total weight of the composition, from about 0.5 percent to about 40 percent of zinc oxide.

The composition also may contain an aqueous component including water, glycols such as propylene glycol, glycerin, dipropylene glycol, and mixtures thereof typically in an amount, based upon the total weight of the composition, from about 35 percent to about 95 percent, e.g. from about 65 percent to about 75 percent.

The composition may include other optional ingredients known in the art such as, for example, colorants, fragrances, anti-microbials, preservatives, emollients, conditioners, surfactants, and the like. For example, the composition may include aloe in an about, based upon the total weight of the composition, from about 0.01 percent to about 15 percent, and/or may include vitamins such as tocopherol and/or tocopherol acetate in an amount, based upon the total weight of the composition, from about 0.01 percent to about 5 percent.

Examples of suitable emollients include, but are not limited to PPG-2 isoceteth-20 acetate available from Bernel Chemical Company, Inc under the tradename, "CUPL PIC," and octyldodecyl neopentanoate available from Bernel Chemical Company, Inc. under the tradename, "Elefac I 205," and may be included in an amount, based upon the total weight of the composition, from about 2 percent to about 10 percent.

In one embodiment wherein longer stability is of particular concern, the composition further contains, based upon the total weight of the composition, from about 0.01 percent to about 5 percent, e.g. from about 0.05 percent to about 2 percent of a structurant. By "structurant," it is meant any compound that enhances the overall body of a product. This effect may be exhibited as an increase in the viscosity and/or stability of a product. Structurants can be water-soluble or oil-soluble, synthetic or natural materials. Water-soluble structurants enhance product viscosity and or stability via swelling in the aqueous phase of a product, thereby "structuring" the aqueous phase. Oil-soluble structurants enhance product viscosity and or stability via associating with micelles and forming bridges or networks between micelles, thereby "structuring" via linkage of micelles. Non-limitiing examples of suitable water-soluble, structurants include gums such as xanthan gum, modified celluloses, and carbomers, e.g. cross-linked polyacrylates such as those available from Noveon, Incorporated under the tradename, "Ultrez-10". Non-limiting examples of suitable oil-soluble, structurants include fatty alcohols such as cetyl alcohol, stearyl alcohol, etc; waxes such as beeswax, caranuba, etc; butters such as shea butter, cocoa butter, etc; and glyceryl esters such as glyceryl stearate.

The composition of the present invention is "sprayable," which means that it is in a liquid form at 20 °C and may be applied to the skin via a conventional hand-held pump sprayer or via a conventional pressurized spray device containing propellant using normal finger pressure. As used herein, "spray" shall mean a jet of finely divided liquid composition. Thus, in embodiments using solid particles, the particles must be of a sufficiently small size that can pass through the tubes and spray orifices used in conventional spraying devices, i.e., for e.g., from about 0.05 µm to about 500 µm.

The composition of the present invention must also possess a rheology that is appropriate for spray dispensers, i.e., it must have a sufficiently low enough viscosity under shear. While the viscosity of the composition may vary depending upon, for example, the skin protectant(s) selected and the size of the spray device components, the viscosity typically may range from about 2000 cps to about 18,000 cps as measured by a Brookfield DV-I+ Viscometer using an LVT #3 spindle and speed of 6 rpm under temperature conditions of about 25 ° C.

The compositions of the present invention may be in a form suitable for application to the skin in either a leave-on product or a rinse-off product.

Another embodiment of the present invention is directed to methods for treating an affected area, e.g. an area affected by dermatitis/diaper rash/ skin rash, using an effective amount of the compositions described above.

Although the "effective amount" of composition used to treat the rash will depend upon, for example, the severity of skin irritation at the affected area and/or the skin protectant agent selected, typically for treating purposes, from about 0.1 mg/square cm to about 3 mg/square cm , for example from about 1 mg/ square cm to about 2.5 mg/ square cm of the composition, is applied to the affected area during treatment.

Beneficially, the compositions of the present invention may be sprayed to the affected area of the skin without the need of having a hand touch the affected area. Further, because the compositions are substantially free of oils and silicones, any safety concerns associated with its possible inhalation are significantly reduced.

The invention illustratively disclosed herein suitably may be practiced in the absence of any component, ingredient, or step which is not specifically disclosed herein. Several examples are set forth below to further illustrate the nature of the invention and the manner of carrying it out. However, the invention should not be considered as being limited to the details thereof.

### Examples

### Example 1: Preparation of Zinc Oxide - Containing Dispersion

A oil in water dispersion comprised of the below components was prepared:

| *Tradename* | *CTFA Name* | *Supplier* | *Amount used (grams)* |
|---|---|---|---|
| Deionized water | Purified water | --- | 233.00 |
| Laponite XLG | Sodium magnesium silicate | Southern Clay Products | 7.50 |
| Keltrol 1000 | Xanthan gum | C.P. Kelco | 1.00 |
| Hetester PHA | Propylene glycol isoceteth-3 acetate | Bernel Chemical Company, Inc. | 50.00 |
| Hexylene glycol | Hexylene glycol | --- | 10.00 |
| Methyl paraben | Methyl paraben | --- | 1.50 |
| Propyl paraben | Propyl paraben | --- | 0.50 |
| Versene NA | Disodium EDTA | Dow Chemical | 1.50 |
| Zinc oxide Usp | Zinc oxide | --- | 65.00 |
| CUPL PIC | PPG-2 Isoceteth-20 Acetate | Bernel Chemical Company, Inc | 10.00 |
| Elefac I 205 | Octyldodecyl Neopentanoate | Bernel Chemical Company, Inc | 40.00 |
| Brij 721 | Steareth-21 | Uniqema | 10.00 |
| NEOBEE M-5 cosmetic | Triglycerides mixed decanotae and octanoate | Stepan Company | 25.00 |
| Luviset PUR | Polyurethane - 1 | BASF Corporation | 45.00 |

### A. Preparation of water phase:

Sodium magnesium silicate was added into a beaker containing 233 g of purified water with rapid mixing. As the temperature of the resulting dispersion was increasing to about 65 °C, the following ingredients were added thereto independently with slow mixing until each respective resulting mixture was homogenous: xanthan gum; propylene glycol isoceteth-3 acetate; hexylene glycol; preservatives; and disodium EDTA.

### B. Preparation of oil phase

PPG-2 isoceteth-20 acetate, octyldodecyl neopentanoate, and triglycerides mixed with decanoate and octanoate were added to a beaker with mixing, then the resulting mixture was heated to about 65 °C with mixing. When these ingredients were melted, the steareth-21 was added thereto with mixing. After the steareth-21 was melted, the zinc oxide was added thereto with mixing until it was dispersed.

### C. Preparation of Oil in Water Dispersion

After the water phase mixture and the oil phase mixture, respectively, reached a temperature of about 65 °C, the oil phase mixture was added to the water phase mixture with rapid mixing. The resulting dispersion was cooled to about 20 °C, then the polyurethane-1 was added thereto. After citric acid (20% solution) was added thereto in order to adjust the final pH to 7.0, the remaining water was added thereto with mixing until homogenous.

The viscosity of the resulting composition was about 15,550 cps as measured by a Brookfield DV-I+ Viscometer using a # 4 spindle and speed of 12 rpm under temperature conditions of about 25° C.

### Example 2: Preparation of Zinc Oxide - Containing Dispersion With Rinse-Off Resistant Agent

A oil in water dispersion comprised of the below components was prepared:

| *Tradename* | *CTFA Name* | *Supplier* | *Amount used*(g) |
|---|---|---|---|
| Deionized water | Purified water | --- | 400.00 |
| Laponite XLG | Sodium magnesium silicate | Southern Clay Products | 11.26 |
| Keltrol 1000 | Xanthan gum | C.P. Kelco | 1.52 |
| Hetester PHA | Propylene glycol isoceteth-3 acetate | Bernel Chemical Company, Inc | 75.19 |
| Hexylene glycol | Hexylene glycol | --- | 15.11 |
| Methyl paraben | Methyl paraben | --- | 2.26 |
| Propyl paraben | Propyl paraben | --- | 0.77 |
| Versene NA | Disodium EDTA | Dow Chemical | 2.25 |
| Zinc oxide Usp | Zinc oxide | --- | 97.54 |
| CUPL PIC | PPG-2 Isoceteth-20 Acetate | Bernel Chemical Company, Inc | 15.21 |
| Elefac I 205 | Octyldodecyl Neopentanoate | Bernel Chemical Company, Inc | 60.49 |
| Brij 721 | Steareth-21 | Uniqema | 15.10 |
| NEOBEE M-5 cosmetic | Triglycerides mixed decanoate and octanoate | Stepan Company | 37.56 |
| Performa V 1608 Polymer | C30-38 olefin isopropyl maleate/MA copolymer | New Phase Technologies | 22.51 |
| Sodium hydroxide | Sodium hydroxide | --- | 0.17 |

### A. Preparation of water phase:

Sodium magnesium silicate was added into a beaker containing 400 g of purified water with rapid mixing. As the temperature of the resulting dispersion was increasing to about 80 °C, the following ingredients were added thereto independently with slow mixing until each respective resulting mixture was homogenous: xanthan gum; propylene glycol isoceteth-3 acetate; hexylene glycol; preservatives; and disodium EDTA.

### B. Preparation of oil phase

PPG-2 isoceteth-20 acetate, octyldodecyl neopentanoate, C30-38 olefin isopropyl maleate/maleic anhydride copolymer and triglycerides mixed decanoate and octanoate were added to a beaker with mixing, then the resulting mixture was heated to about 80°C with mixing. When these ingredients were melted, the steareth-21 was added thereto with mixing.
After the steareth-21 was melted, the zinc oxide was added thereto with mixing until it was dispersed.

### C. Preparation of Oil in Water Dispersion

After the water phase mixture and the oil phase mixture, respectively, reached a temperature of about 80 °C, the oil phase mixture was added to the water phase mixture with rapid mixing. The resulting dispersion was cooled to about 20 °C, then the sodium hydroxide (20% solution) was added thereto in order to adjust the final pH to 7.0, the remaining water was added thereto with mixing until homogenous.

The viscosity of the resulting composition was about 2070 cps as measured by a Brookfield DV-I+ Viscometer using a # 3 spindle and speed of 12 rpm under temperature conditions of about 25° C.

### Example 3: Rinse-Off Resistance Test

The composition prepared in accordance with Example 1 was added to a conventional hand-held pump sprayer with model number 24-410 available from Sequist under the tradename, "Eurolock". Approximately 0.5 grams (3 spray pumps) of the composition was then applied to about a 2 inch by 3 inch area of the volar forearm. After allowing the product to dry for about 1 minute, the arm was placed under slowly running tap water for about 15 seconds. Rinse-off resistance was measured visually by evaluating whiteness (e.g. zinc oxide residue) of skin.

This method was repeated, but with using the composition prepared in accordance with Example 2.

A product with acceptable rinse-off resistance retained at least about 60% of original whiteness on skin.

This Example showed that the composition of Example 2, which contained a rinse-off resistant agent, possessed an acceptable rinse-off resistance and thus adhered to the skin more than the composition of Example 1, which did not possess an acceptable rinse-off resistance.

### Example 4: Transfer Resistance Test

The composition prepared in accordance with Example 1 was added to the conventional hand-held pump sprayer of Example 3. In order to measure the transfer of product to clothing or diaper, approximately 0.5 grams (3 Spray pumps) of the composition was then applied to about a 2 inch by 3 inch area of the volar forearm. After allowing the product to dry for about 1 minute, a dry two-ply paper towel was placed over the treated area. The paper towel was then wetted with a water spray until damp and remained on the treated area for about 3 minutes with gentle patting. The paper towel was then removed from the treated area and evaluated visually for whiteness (e.g. zinc oxide residue) of skin.

This method was repeated, but with using the composition prepared in accordance with Example 2.

A product with acceptable transfer resistance retained at least about 75% of original whiteness on skin.

This Example showed that the composition of Example 2, which contained a rinse-off resistant agent, possessed an acceptable transfer resistance and thus transferred less zinc oxide from skin to paper towel than the composition of Example 1, which did not possess an acceptable transfer resistance.

### Example 5: Preparation of Zinc Oxide - Containing Dispersion With Rinse-Off Resistant Agent

A oil in water dispersion comprised of the below components was prepared:

| *Tradename* | *CTFA Name* | *Supplier* | *Amount used*(g) |
|---|---|---|---|
| Deionized water | Purified water | --- | 517.120 |
| Bentone EWCG | Hectorite | Elementis Specialties | 6.40 |
| Keltrol 1000 | Xanthan gum | C.P. Kelco | 0.40 |
| DexPanthenol | Panthenol | Roche Vitamins / Hoffman LaRoche Inc. | 0.80 |
| Hexylene glycol | Hexylene glycol | --- | 16.00 |
| Methyl paraben | Methyl paraben | --- | 3.2 |
| Propyl paraben | Propyl paraben | --- | 1.6 |
| Versene NA | Disodium EDTA | Dow Chemical | 0.80 |
| Zinc oxide Usp | Zinc oxide | --- | 104.00 |
| CUPL PIC | PPG-2 Isoceteth-20 Acetate | Bernel Chemical Company, Inc | 24.00 |
| Multiwax 445 | Microcrystalline wax | --- | 8.00 |
| Brij 721 | Steareth-21 | Uniqema | 24.00 |
| NEOBEE M-5 cosmetic | Triglycerides mixed decanoate and octanoate | Stepan Company | 60.00 |
| Performa V 1608 Polymer | C30-38 olefin isopropyl maleate/MA copolymer | New Phase Technologies | 16.00 |
| phenoxyethanol | | | 5.6 |
| Vitamin E Acetate | Tocopherol acetate | --- | 0.08 |
| Lexgard GMCy | Glyceryl caprylate | Inolex Chemical Company | 12.00 |

### A. Preparation of water phase:

The hexylene glycol, methyl paraben, and propyl paraben were combined in a beaker to form an initial pre-mixture.

The hectorite was added to an independent beaker containing 517.12 g of purified water with rapid mixing for about 20 minutes under ambient conditions in order to disperse the hectorite. The xanthan gum was then added thereto with mixing until dispersed. As the temperature of the resulting dispersion was increasing to about 80 °C, the following ingredients were added thereto independently with slow mixing until each respective resulting mixture was homogenous: the initial pre-mixture; disodium EDTA; and glyceryl caprylate.

### B. Preparation of oil phase

PPG-2 isoceteth-20 acetate and caprylic/capric triglycerides were added to a beaker with mixing, then the resulting mixture was heated to about 80°C with mixing until the former melted. As the temperature of the resulting dispersion was increasing to about 80 °C, the following ingredients were added thereto independently with slow mixing until each respective resulting mixture was homogenous: steareth-21, C30-38 olefin isopropyl maleate/maleic anhydride copolymer, and zinc oxide.

### C. Preparation of Oil in Water Dispersion

After the water phase mixture and the oil phase mixture, respectively, reached a temperature of about 80 °C, the oil phase mixture was added to the water phase mixture with rapid mixing. After the resulting dispersion was cooled to about 40 °C, the panthenol (preheated to a temperature of about 40°C) was added thereto. After the phenoxyethanol was added thereto with mixing until homogeneous, pH was measured and found to be about 7.0. The remaining water was then added thereto with mixing until homogenous.

The viscosity of the resulting composition was about 20,000 cps as measured by a Brookfield DV-I+ Viscometer using a # 4 spindle and speed of 3 rpm under temperature conditions of about 25° C.

## Claims

1. A skin-protectant composition comprising:
a. a suspending agent;
b. a rinse-off resistant agent; and
c. at least one skin protectant agent,
wherein the composition is substantially free of oil and silicone and is spray pumpable.

2. The composition of claim 1, wherein the suspending agent is a clay selected from the group consisting of pyrophillite, talc, and mixtures thereof.

3. The composition of claim 1, wherein the suspending agent is a clay selected from the group consisting of smectite, sepiolite, zeolite, palygorskite, and mixtures thereof.

4. The composition of claim 3 wherein the smectite clay is an aluminosilicate selected from the group consisting of bentonite, montmorillonite, hectorite, sucinite, saponite, nontronite, vermiculite, beidellite, stevensite and synthetically made counter parts and mixtures thereof.

5. The composition of claim 4 wherein the clay is montmorillonite.

6. The composition of claim 1 wherein the suspending agent is a clay adduct prepared by treating a clay with a quaternary ammonium compound that binds to the clay, the quaternary ammonium compound having the structure:
R₁R₂R₃R₄N+
wherein
R₁, R₂, R₃ and R₄ are each independently selected from the group consisting of H, an alkyl having from about 1 to about 22 carbon atoms, an alkenyl having from about 1 to about 22 carbon atoms, and an aralkyl having from about 1 to about 22 carbon atoms, provided that at least one of the R groups is an alkyl having from about 1 to about 22 carbon atoms, an alkenyl having from about 1 to about 22 carbon atoms, or an aralkyl having from about 1 to about 22 carbon atoms.

7. The composition of claim 1, wherein the rinse-off resistant agent is selected from the group consisting of maleic anyhydride terpolymers, polyvinylpyrillidone, and copolymers and mixtures thereof.

8. The composition of claim 1, wherein the rinse-off resistant agent is selected from C30-38 olefin isopropyl maleate/maleic anhydride copolymer, vinylpyrrolidone/eicosene copolymer and copolymers and mixtures thereof.

9. The composition of claim 1, wherein the skin protectant agent is selected from the group consisting of oatmeal, soy, mica, silica, titanium dioxide, hydrocortisone, benzoyl peroxide, topical starch, zinc oxide, aluminum hydroxide gel, allantoin, cocoa butter, zinc acetate, calamine, glycerin, kaolin, talc, zinc carbonate, and mixtures thereof.

10. The composition of claim 1, wherein the skin protectant agent is selected from the group consisting of oatmeal, soy, zinc oxide, and mixtures thereof.

11. The composition of claim 1 containing, based upon the total weight of the composition,
from greater than about 0.1 percent and less than about 5 percent of the suspending agent;
from greater than about 0.1 percent and less than about 10 percent of the rinse-off resistant agent;
from greater than about 0.001 percent and less than about 40 percent of the skin protectant agent.

12. The composition of claim 11 further comprising, based upon the total weight of the composition, from greater than about 0.01 percent and less than about 5 percent of a structurant.

13. The composition of claim 1 containing, based upon the total weight of the composition,
from greater than about 0.1 percent and less than about 2 percent of the suspending agent;
from greater than about 1 percent and less than about 5 percent of the rinse-off resistant agent;
from greater than about 0.01 percent and less than about 5 percent of the skin protectant agent.

14. The composition of claim 1 containing, based upon the total weight of the composition,
from greater than about 0.1 percent and less than about 5 percent of a clay suspending agent;
from greater than about 0.1 percent and less than about 10 percent of the rinse-off resistant agent selected from C30-38 olefin isopropyl maleate/maleic anhydride copolymer, vinylpyrrolidone/eicosene copolymer, and copolymers and mixtures thereof; and
from greater than about 0.001 percent and less than about 40 percent of the skin protectant agent selected from zinc oxide, oatmeal, soy, and mixtures thereof.

15. A hand-held spray dispenser containing the composition of claim 1.

16. The use of a composition comprising, based upon the total weight of the composition,
from greater than about 0.1 percent and less than about 5 percent of the suspending agent;
from greater than about 0.1 percent and less than about 10 percent of the rinse-off resistant agent;
from greater than about 0.5 percent and less than about 10 percent of the skin protectant agent,
wherein the composition is sprayable and substantially free of oil and silicone, in the treatment of diaper rash.
